# EUROPEAN PATENT SPECIFICATION

(11) **EP 2 187 871 B1**
(45) Date of publication and mention of the grant of the patent: **13.04.2016**
(21) Application number: 07802671.3
(22) Date of filing: 16.08.2007
(51) Int. Cl.: A61K 9/20, A61K 9/08, A23G 3/34, A23G 4/06, A23L 27/20, A61K 8/37, A61K 8/42, A61Q 11/00

(54) **MIXTURES COMPRISING PELLITORIN AND USES THEREOF**
PELLITORIN ENTHALTENDE GEMISCHE UND IHRE VERWENDUNGEN
MÉLANGES CONTENANT DE LA PELLITORINE ET LEURS UTILISATIONS

(43) Date of publication of application: 26.05.2010
(73) Proprietor: Symrise AG, 37603 Holzminden (DE)
(72) Inventor: MACHINEK, Arnold, 37603 Holzminden (DE); WÖHRLE, Ingo, 28203 Bremen (DE); LEY, Jakob, 37603 Holzminden (DE); KINDEL, Günter, 37671 Höxter (DE)
(74) Representative: Fabry, Bernd
(86) International application number: PCT/EP2007/058545
(87) International publication number: WO 2009/021558

(56) References cited:
- EP-A- 1 913 976
- WO-A-2005/044778
- WO-A-2008/049520
- AU-B2- 439 878
- US-A1- 2004 241 312
- US-A1- 2007 036 838

## Description

### FIELD OF INVENTION

The present invention relates to products and mixtures comprising (a) pellitorin and (b) selected cooling agents. The present invention further relates to products and medica-ments for soothing irritated oral and/or nasal tissues. The present invention also relates to the use of such mixtures for soothing irritated oral and/or nasal tissues and the use of such mixtures for reducing bitterness, in particular the bitterness of oral care products, confectionery products and medicaments, more specifically reducing the bitterness of menthol and menthol containing products and medicaments.

### BACKGROUND OF THE INVENTION

Many examples exist of medicaments designed to relieve or sooth irritated oral and nasal tissues. These medicaments typically rely upon a pharmaceutically active ingredient such as benzocaine which is a local anaesthetic. These ingredients, whilst on the whole successful, are sometimes not able to effectively sooth the nasal tissues and the nasopharyngeal region. Furthermore, the effects of pharmaceutically active ingredients such as these are sometimes considered by consumers as being more than is necessary to achieve the required results. A need exists for medicaments that provide a soothing effect in a moderate manner.

Soothing of irritated oral and nasal tissues may occur via increased moisturization of the tissues. However, many ingredients suitable for increasing moisturization of the oral and nasal tissues have noted drawbacks. For example, citric acid is able to increase oral salivation around the area of the tongue; yet is not able to improve moisturization in the entirety of the oral cavity and does not modulate nasal moisturization. This tends to prevent citric acid from being able to provide notable soothing effect in the naso-phayngeal area. Furthermore, levels of citric acid that are capable of producing noticeable benefits around the tongue may taste acidic or astringent, and may also negatively impact product stability due to the high hy-groscopicity of citric acid.

Further, a need exists to provide medicaments that successfully sooth irritated oral and/or nasal tissues without associated aesthetic negatives and/or product stability issues, preferably in addition being able to reduce the bitterness of oral care products, confectionery products and medica-ments, in particular the bitterness of menthol.

US 5,372,824 teaches a mint flavoured chewing gum having reduced bitterness comprising a mint flavour agent from which at least a portion of I- menthol has been removed. In an embodiment, the mint flavoured chewing gum includes a cooling agent, for example menthyl lactate. The cooling agent can comprise, for example 0.1 % to 5.0 % by weight of the reduced mint oil. Details of a peppermint fraction after menthol reduction, which is free of menthyl lactate, are also disclosed. Pellitorin is not mentioned.

US 2004/0018954 discloses mixtures containing menthol and menthyl lactate, characterized in that it comprises menthol and menthyl lactate in a ratio by weight in the range of 1: 4 to 4: 1 and the corresponding crystallization point is below room temperature of 25 °C. Such a composition which is liquid at room temperature was produced by stirring menthol and menthyl lactate in solid form within the mentioned ratio without heating, i.e. without melting the components. Pellitorin is not mentioned.

WO 2004/000787 describes a method for producing 2E,4Z-decadienoic acid-N-isobutylamide (cis-pellitorin) and the use thereof as a pungent agent and a flavouring that generates heat, preferably in foodstuffs, oral hygiene or gourmet preparations. Menthol is not mentioned, nor specific cooling agents.

US 2004/0241312 concerns the use of 2E,4E-decadienoic acid-N- isobutylamide (trans-pellitorin) as flavourant, in particular as salivation inducing flavour substance, preferably in a foodstuff or nutrient, an oral hygienic preparation or a gourmet or snack preparation. Further, US 2004/0241312 concerns preparations, semi-finished preparations as well as fragrance, aroma and taste compositions, containing trans-pelletorin as well as a processes for the production of trans-pelletorin. Menthol is not mentioned, nor specific cooling agents.

US 2005/0075368 describes mixtures of at least four conjugated C₁₀-C₁₁-2E,4E-alkadienamides, obtainable synthetically or from a dried, ground Piper species, Piper longum Linn or Piper peepuloides, having flavour and sensory attributes in the oral cavity and on skin. Flavours like peppermint oil are mentioned, menthol is not mentioned, nor are specific cooling agents.

WO 2006/039945 relates to composition comprising menthyl lactate, neo- menthol and menthol, optionally further comprising neoisomenthol and/or isomenthol, wherein said composition has a solidification point below +5 Degrees C. Pellitorin is not mentioned.

US 2006/0204551 provides a salivation cocktail that comprises a food acid and a tingling sensate, the combination of a food acid and a tingling sen-sate synergistically increasing salivation, one of the tingling sensates mentioned is 2E,4E-decadienoic acid-N-isobutylamide (trans-pellitorin). In one embodiment of US 2006/0204551, a cough drop or lozenge is described containing a) menthol or other medicaments for the treatment of sore throat, coughing or other upper respiratory ailments and b) one of the salivation cocktails as described in US 2006/0204551. Peppermint oil is not mentioned, nor are cooling agents.

US 2007/0036838 teaches the use of certain salivating agents in the preparation of a medicament for soothing irritated oral and/or nasal tissues is provided. US 2007/0036838 further relates to compositions comprising certain salivating agents and a cooling agent selected from the group consisting of menthol, peppermint oil, N-substituted-p-menthane-3-carboxamides, acyclic tertiary and secondary carboxamides, 3-(I-menthoxy)propan-1,2-diol, monomenthyl glutarate and mixtures thereof. The amount of salivating agents according to US 2007/0036838 preferably is at least 100 ppm and most preferably lies in the range from about 0.02 wt. % (= 200 ppm) to about 1 weight %, based on the total weight of the medicament. In the examples given, the final concentration of trans-pellitorin in the ready-to-use products is 800 ppm.

EP 1 774 956 describes oral and dental hygiene and cleansing products that contain, based on their weight, a) at least 40 % of at least one polyol selected from the group sorbitol, glycerin and/or 1,2-propylene glycol, and b) at least one salivation-promoting substance. Cis- and trans-pellitorin are mentioned as being preferred salivation-promoting substances. EP 1 774956 mentions peppermint oil as a possible flavouring agent, menthol is not mentioned. The only cooling agent mentioned is N-ethyl-p-menthane-3- carboxamide (WS-3), EP 1 774 956 lists WS-3 as a warming / hot spicy substance.

WO 2007/068403 describes oral and dental hygiene and cleansing products that have a mineralizing and/or remineralizing effect if they contain, based on their weight, a) 0.005 to 10 % of at least one calcium salt, and b) 0.005 to 10 % of at least one salivation-promoting substance. Cis- and trans-pellitorin are mentioned as being preferred salivation-promoting substances. WO 2007/068403 mentions peppermint oil as a possible flavouring agent and menthol as a possible active ingredient in chewing gums. The only cooling agent mentioned is N-ethyl-p-menthane-3-carboxamide (WS- 3), WO 2007/068403 lists WS-3 as a warming / hot spicy substance.

### SUMMARY OF THE INVENTION

The present invention provides mixtures and products that effectively sooth irritated oral and/or nasal tissues and can reduce bitterness. The products described herein incorporate a mixture comprising (a) pellitorin and (b) selected cooling agents, said mixture and product modulating oral and/or nasal secretions, providing relief to a consumer's oral and/or nasal tissues, in addition having reduced the bitterness.

The invention relates to a mixture comprising:
(a) pellitorin, and
(b) one or more physiological cooling agents selected from the group consisting of:
   N-[[5-methyl-2-(1-methylethyl)cyclohexyl]-carbonyl]glycine ethyl ester, menthyl-3-hydroxy butyrate, menthyl-3-oxo butyrate, menthyl-3-oxo pentanoate, menthyl lactate, menthone glycerine acetal, menthol glycol carbonate, menthol propyleneglycol carbonate and menthol glycerin carbonate;
   and optionally one or more compounds selected from the groups (c), (d) and/or (e):
(c) menthol and/or peppermint oil or mixtures thereof;
(d) one or more additional physiological cooling agents selected from the group consisting of: N-substituted-p-menthane-3-carboxamides, acyclic tertiary and secondary carboxamides, 3-(I-menthoxy)propan-1,2-diol, monomenthyl glutarate, monomenthyl succinate or its salts,
(e) one or more adjuvants selected from the group consisting of glycerin, ethylene glycol, 1,2-propylene glycol, diethyl malonate and/or dibutyl malonate
with the proviso that
(i) a first mixture comprising (in parts per weight)

| | |
|---|---|
| L-N°-(menthancarboxyl)glycin-N-ethylamid | 15 |
| Solution of 10 wt.-% pellitorin in propyleneglycol/peppermint oil (1:1); | 10 |
| L-Menthyllactate | 65 |
| O-L-Menthyl-O'(2-hydroxyethyl)carbonate | 10 |

and
(ii) a second mixture comprising (in parts per weight)

| | |
|---|---|
| Spearmint oil | 10.57 |
| Peppermint oil piperita (menthol content approx.. 53 % b.w.) | 43.44 |
| Sucralose | 1.73 |
| Saccharin | 2.61 |
| Triethyl citrate | 0.10 |
| L-Menthol, natural | 15.59 |
| Triacetine (glycerol triacetate) | 0.07 |
| Benzyl alcohol | 0.07 |
| Capsicum extract 1 million Scoville | 0.07 |
| Ethanol | 5.03 |
| 2-Hydroxyethyl menthyl carbonate | 3.19 |
| L-Menthyl methyl ether | 3.10 |
| Piperine | 0.01 |
| Cis/trans Pellitorin (20/80) (m/m) | 0.01 |
| Plant oil | 4.50 |
| D-Limonene | 2.16 |
| Anethole | 7.75 |

are excluded.

### DETAILED DESCRIPTION OF THE INVENTION

Unless otherwise stated herein, all %ages are weight %ages. Unless otherwise stated herein, all measurements are taken at 20 degrees centigrade.

The present invention provides the use of a mixture comprising or consisting of (a) pellitorin and (b) selected cooling agents in the preparation of a product able to sooth irritated oral and/or nasal tissues by modulating oral and/or nasal secretion. As used herein, "oral" includes the buccal cavity, tongue and the throat, and "nasal" includes the nose, nasal cavity and the nasopharynx. As used herein, "soothing" includes relaxing, moisturizing, relieving, reducing pain and the like. Preferably, the product sooths irritated oral and nasal tissues by modulating oral and nasal secretions. Oral and nasal secretion includes salivation, moisturization and mucosal secretion, preferably in the nasal passage, nasal conchae, pharynx, nasopharynx, oral cavity, soft palate and the tongue.

### Part (a) of a mixture according to the present invention

In the context of the present invention pellitorin means 2,4-decadienoic acid-N-isobutylamide, wherein the two carbon-carbon - double bonds independently can have (E)-or (Z)-configuration. Thus, in the context of the present invention pellitorin means 2E,4E-decadienoic acid-N-isobutylamide (trans-pellitorin), 2E,4Z-decadienoic acid-N-isobutylamide (cis-pellitorin),

2Z,4Z-decadienoic acid-N-isobutylamide, 2Z,4E-decadienoic acid-N- isobutylamide, and mixtures thereof.

Preferred in the present invention are trans-pellitorin and/or cis-pellitorin and mixtures thereof, in particular mixtures wherein the weight ratio of trans-: cis-pellitorin is in the range of 40: 1 - 1: 1, more preferably in the range of 30: 1-5:1, most preferably in the range of 25: 1 - 10: 1.

Methods of obtaining pellitorin suitable for use herein are described in WO 2004/000787 and US 2004/0241312.

### Part (b) of a mixture according to the present invention

The physiological cooling agents of part (b) are selected from the group consisting of:

N-[[5-methyl-2-(1-methylethyl)cyclohexyl]-carbonyl]glycine ethyl ester, menthyl-3-hydroxy butyrate, menthyl-3-oxo butyrate, menthyl-3-oxo pen- tanoate, menthyl lactate, menthone glycerine acetal, menthol glycol carbonate, menthol propyleneglycol carbonate and menthol glycerin carbonate and mixtures thereof.

N-[[5-methyl-2-(1-methylethyl)cyclohexyl]-carbonyl]glycine ethyl ester, is also known as WS-5, as for example described in US 7,189,760, also commercially available from Mille-nium; menthyl-3-hydroxy butyrate, preferably l-menthyl-3-hydroxy butyrate, as described in FR 2 577 922; menthyl-3-oxo butyrate and menthyl-3-oxo pentanoate are known from FR 2 577 922 and DE 38 16 360 respectively, the use of these compounds as cooling agents is described in US Prov. 60/883,400 (Symrise GmbH AND Co. KG) and patent applications claiming its priority; menthyl lactate, preferably l-menthyl lactate, more preferably I-menthyl I- lactate, as described in DE 26 08 226, for example commercially available as Frescolat^{(R)} ML from Symrise; in a preferred embodiment of the present invention natural I-menthyl-lactate is used (cf. Flavour Frag. J. 2006, 21, 725-730); menthone glycerine acetal, preferably l-menthone glycerine acetal as described in US 5,266,592, for example commercially available as Frescolat^{(R)} MGA from Symrise; menthol glycol carbonate, menthol propyleneglycol carbonate and menthol glycerin carbonate as described in US 5,703,123; preferred are l-menthol glycol carbonate (O-L-Menthyl-O'-(2-hydroxyethyl) carbonate), also known as Frescolat^{(R)} MGC from Symrise, and l-menthol propylene glycol carbonate, also known as Frescolat^{(R)} MPC from Symrise.

I-menthyl lactate:

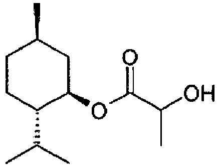

I-menthol glycol carbonate:

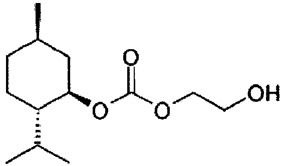

I-menthol propylene glycol carbonate, preferably a mixture of isomers:

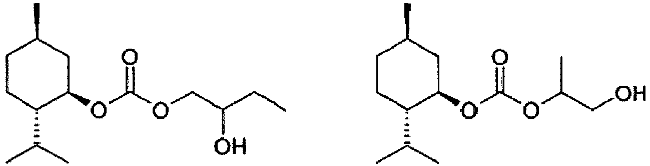

l-menthone glycerine acetal:

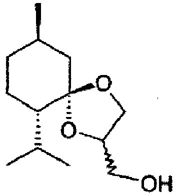

Optional Part (c) of a mixture according to the present invention

Part (c) consists of menthol and/or peppermint oil or mixtures thereof.

In the context of the invention, the menthol preferably is d-menthol, I- menthol or any desired mixture thereof, l-menthol, d-menthol and racemic menthol being preferred and I-menthol being particularly preferred.

Peppermint oils are understood as meaning specifically the essential (i.e. obtained by means of steam distillation) oils of certain Mentha species, in particular from Mentha arvensis (field mint, also called cornmint in US language) and from Mentha piperita (called peppermint in US language), which include Mentha piperita oils with names of regional origin from specific cultivation areas, such as Willamette, Yakima and Madras.

### Optional Part (d) of a mixture according to the present invention

The optional physiological cooling agents of part (d) are selected from the group consisting of:

N-substituted-p-menthane-3-carboxamides, acyclic tertiary and secondary carboxamides, 3-(I-menthoxy)propan-1,2-diol (Coolant Agent 10 by Taka- sago), monomenthyl glutarate, monomenthyl succinate or its salts, and mixtures thereof.

The carboxamides found most useful are those described in US 4,136,163, and US 4,230,688. The carboxamides in US 4,136,163 are N-substituted-p- menthane-3-carboxamides, such as N-ethyl-p-menthane-3-carboxamide, commercially available as WS-3 from Wilkinson Sword. The carboxamides of US 4,230,688 are certain acyclic tertiary and secondary carboxamides, such as trimethyl isopropyl butanamide, commercially available as WS-23 from Wilkinson Sword. More preferred for use herein are monomenthyl glutarate, monomenthyl succinate, N-ethyl-p-menthane-3-carboxamide, trimethyl isopropyl butanamide and mixtures thereof.

Monomenthyl glutarate is commercially available as Ultracool 2 from IFF (Netherlands), monomenthyl succinate, or its alkali metal or alkaline earth metal salts, are described WO 97/07771 and commercially available from Mane Fils.

When present, these optional physiological cooling agents of part (d) can improve the soothing effect on irritated oral and/or nasal tissues of a mixture according to the present invention.

### Optional Part (e) of a mixture according to the present invention

The optional adjuvants of part (e) are selected from the group consisting of: glycerin, ethylene glycol, 1,2-propylene glycol, diethyl malonate, dibutyl malonate and mixtures thereof.

When present, these adjuvants can improve the soothing effect on irritated oral and/or nasal tissues and enhance the bitterness reducing effect of a mixture according to the present invention. In addition these adjuvants improve solubilization and stabilization of pellitorin of part (a) in said mixtures.

If a formulation (e.g. mixture, flavour composition, product or medicament) according to the present invention comprises pellitorin, propylene glycol, peppermint oil, L-Menthyl lactate and O-L-Menthyl-O'-(2-hydroxyethyl) carbonate, then the ratio by weight of pellitorin: L-Menthyl lactate: O-L- Menthyl-O'-(2-hydroxyethyl) carbonate (Frescolat^{(R)} MGC from Symrise) is not 1: 65:10.

Such mixtures have already been described in still unpublished PCT/EP2007/055157 (Symrise GmbH AND Co. KG), US Prov. 60/829,953 (Symrise GmbH AND Co. KG) and US Prov. 60/883,400 (Symrise GmbH AND Co. KG).

Preferred according to the present invention are mixtures comprising :
(a) trans-pellitorin and/or cis-pellitorin, and
(b) one or more physiological cooling agents selected from the group consisting of: N-[[5-methyl-2-(1-methylethyl)cyclohexyl]-carbonyl]glycine ethyl ester, menthyl-3-hydroxy butyrate, menthyl-3-oxo pentanoate, menthyl lactate, menthone glycerine acetal, menthol glycol carbonate and menthol propyle- neglycol carbonate; and
(c) menthol and/or peppermint oil or mixtures thereof; and
(e) one or more adjuvants selected from the group consisting of glycerin, ethylene glycol, 1,2-propylene glycol, diethyl malonate and/or dibutyl malo- nate; and optionally
(d) one or more additional physiological cooling agents selected from the group consisting of N-substituted-p-menthane-3-carboxamides, acyclic tertiary and secondary carboxamides, 3-l-menthoxy propan-1,2-diol, monomenthyl glutarate, monomenthyl succinate.

More preferred according to the present invention are mixtures comprising:
(a) trans-pellitorin, and
(b) one or more physiological cooling agents selected from the group consisting of: N-[[5-methyl-2-(1-methylethyl)cyclohexyl]-carbonyl]glycine ethyl ester, menthyl-3-hydroxy butyrate, menthyl-3-oxo pentanoate, menthyl lactate, menthone glycerine acetal, menthol glycol carbonate and menthol propyle- neglycol carbonate; and
(c) menthol; and
(e) one or more adjuvants selected from the group consisting of glycerin, 1,2-propylene glycol and/or diethyl malonate; and optionally
(d) one or more additional physiological cooling agents selected from the group consisting of N-ethyl-p-menthane-3-carboxamide (WS-3), trimethyl isopropyl butanamide (WS-23), monomenthyl glutarate.

Most preferred according to the present invention are mixtures comprising:
(a) trans-pellitorin and cis-pellitorin; and
(b) one or more physiological cooling agents selected from the group con- sisting of: N-[[5-methyl-2-(1-methylethyl)cyclohexyl]-carbonyl]glycine ethyl ester, menthyl lactate, menthone glycerine acetal, menthol glycol carbonate and menthol propyleneglycol carbonate; and
(c) menthol and peppermint oil; and
(e) 1,2-propylene glycol and/or diethyl malonate; and optionally
(d) N-ethyl-p-menthane-3-carboxamide and/or trimethyl isopropyl butanamide.

Preferably, a mixture according to the present invention comprises from 0.02 % (200 ppm) to 2 % pellitorin, more preferably from 0.05 % (500 ppm) to 1.5 %, most preferably from 0.10 % (1000 ppm) to 1.25 %, in each case based on the total weight of the mixture.

Preferably, the total amount of the cooling agents of part (b) in a mixture according to the present invention is in the range from 5 % to 98 %, preferably from 15 % to 75 %, in each case based on the total weight of the mixture.

The mixtures and products herein can additionally include a flavouring agent. As used herein, the term 'flavouring agent' means those flavour essences and equivalent synthetic ingredients which are added to the flavour composition for the principal purpose of providing flavour to the product. It excludes physiological warming and physiological cooling agents as well as the constituents of parts (a) to (e) of a mixture according to the present invention.

A flavour composition according to the present invention comprises
(i) a mixture according to the present invention as described herein above, and
(ii) one, two, three, four, five, six, seven, eight or more (additional) flavouring agents, said flavouring agents not being part of the mixture (i).

Flavouring agents well known in the art can be added to the flavour compositions of the invention. These flavouring agents can be chosen from synthetic flavouring liquid and/or oils derived from plants leaves, flowers, fruits and so forth, and combinations thereof. Representative flavouring liquids include: artificial, natural or synthetic fruit flavours such as eucalyptus, lemon, orange, banana, grape, lime, apricot and grapefruit oils and fruit essences including apple, strawberry, cherry, orange, pineapple and so forth; bean and nut derived flavours such as coffee, cocoa, cola, peanut, almond and so forth; and root derived flavours such as licorice or ginger.

Preferably, a flavouring composition according to the present invention comprises from 0.01 % (100 ppm) to 1 % pellitorin, more preferably from 0.025 % (250 ppm) to 0.50 % (5000 ppm), most preferably from 0.050 % (500 ppm) to 0.25 % (2500 ppm), in each case based on the total weight of the flavouring composition.

Preferably, a flavouring composition according to the present invention comprises from a total amount of 0.1 % to 15 % of the cooling agents of part (b) of the present invention, more preferably from 0.25 % to 12 %, most preferably from 0.5 % to 8 %, in each case based on the total weight of the mixture or flavouring composition.

A product according to the present invention preferably is a foodstuff, a confectionery product, an oral care product or a medicament.

Generally, a total amount of pellitorin in the range from 0.000055 % to 0.0095 % (corresponds to 0.55 - 95 ppm) can be incorporated into a product according to the present invention. Advantageously, a product according to the present invention comprises a total amount of pellitorin of less than 80 ppm, preferably in the range from 0.000055 % to 0.0075 % (corresponds to 0.55 - 75 ppm), more preferably in the range from 0.000075 % to 0.005 % (corresponds to 0.75 - 50 ppm), even more preferably in the range from 0.00009 % to 0.0025 % (corresponds to 0.9 - 25 ppm), most preferably in the range from 0.0001 % to 0.00175 % (corresponds to 1 - 17.5 ppm), in each case based on the total weight of the product.

The amount of flavouring employed in a product according to the present invention is normally a matter of preference subject to such factors as flavour type, base type and strength desired. In general, amounts of flavouring composition according to the present invention of up to 4 % by weight are usable with amounts of from 0.1 % to 1.50 % of flavouring composition according to the present invention being preferred, in each case based on the total weight of the product.

The mixtures or flavouring compositions according to the present invention described herein above can be added to, for example, compositions for the preparation of carbonated fruit beverages, carbonated cola drinks, wine coolers, cordials, flavoured water, powders for drinks (e.g., powdered sports or "hydrating" drinks), hard candy, soft candy, taffy, chocolates, sugarless candies, chewing gum, bubble gum, condiments, spices and seasonings, dry cereal, oatmeal, granola bars, alcoholic beverages, energy beverages, juices, teas, coffees, salsa, gel beads, film strips for halitosis, gelatin candies, pectin candies, starch candies, lozenges, cough drops, throat lozenges, throat sprays, toothpastes and mouth rinses. Use levels of mixtures and of flavouring composition according to the present invention and relative amounts its constituents can be adjusted by persons of ordi- nary skill in the art depending of the flavour of the additives employed in the end use food or beverage, or the taste or flavour of the end product itself.

It was further found that mixtures and flavouring compositions according to the present are able to reduce the bitterness of confectionery, foodstuffs, oral care or medicaments, for example the when incorporated into chewing gum bases (which often taste bitter), confectionery or toothpaste bases having bitter notes, products containing sweeteners having bitter taste or after-taste, or the bitter taste of menthol, which is a known disadvantage of menthol, in particular at higher dosage levels.

It is also important to suppress the bitter taste of many pharmaceutical active ingredients, because the willingness of the patient, in particular of patients who are sensitive to bitterness, such as children, to take the preparation orally can be markedly increased as a result. Many pharmaceutical active ingredients, for example aspirin, salicin, paracetamol, or quinine, to name but only a few for the purposes of illustration, have a pronounced bitter taste and/or after-taste.

It was further found that mixtures, flavouring compositions and products according to the present invention show a prolonged cooling effect in the oral and/or nasal cavity, upon consumption. Thus, when used in the above indicated (preferred) amounts, a longlasting cooling and/or freshening effect is observed, the cooling intensity of the cooling agents of part (b) generally not being augmented. A "long-lasting" effect means that the cooling and/or and freshening effect is perceived for a longer period of time in terms of taste. Surprisingly, not only a "long-lasting" cooling and/or freshening effect was observed, but additionally a prolonged soothing effect on irritated oral and/or nasal tissues. The "longlasting" cooling and/or freshening effects were particularly observed in the presence of part (c) menthol and/or peppermint oil or mixtures thereof.

Thus, in a further aspect of the present invention, pellitorin is used for prolonging the cooling and/or freshening effect of the cooling agents of part (b) of the present invention, preferably in the presence of part (c) menthol and/or peppermint oil or mixtures thereof.

In addition, it was observed that pellitorin improved and rounded off the flavour profile of flavouring compositions and products according to the present invention. Improved and rounded off flavour profiles were particularly found in the presence of part (c) menthol and/or peppermint oil or mixtures thereof, while at the same time also reducing the bitterness of menthol and/or peppermint oil.

Products according to the present invention may comprise one or more food acids, preferably selected from the group consisting of acetic acid, adipic acid, aspartic acid, benzoic acid, sorbic acid, caffeotannic acid, citric acid, iso-citric acid, fumaric acid, galacturonic acid, glucuronic acid, glyceric acid, glycolic acid, ketoglutaric acid, [alpha]-ketoglutaric acid, lactic acid, lactoisocitric acid, malic acid, oxalacetic acid, oxalic acid, pyruvic acid, quinic acid, shikimic acid, succinic acid, tannic acid, and tartaric acid. In a preferred embodiment, the food acids is selected from citric acid, lactic acid, malic acid, succinic acid, fumaric acid, tartaric acid, ascorbic acid or adipic acid.

The flavouring compositions and products of the present invention may further comprise a physiological warming agent.

Preferred physiological warming agents are those selected from the group consisting of vanillyl alcohol n-butyl ether, vanillyl alcohol n-propyl ether, vanillyl alcohol isopropyl ether, vanillyl alcohol isobutyl ether, vanillyl alcohol n-amino ether, vanillyl alcohol isoamyl ether, vanillyl alcohol n-hexyl ether, vanillyl alcohol methyl ether, vanillyl alcohol ethyl ether, gingerol, shogaol, paradol, zingerone, capsaicin, dihydrocapsaicin, nordihydrocapsaicin, homocapsaicin, homodihydrocapsaicin, iso-propyl alcohol, iso- amylalcohol, benzyl alcohol, eugenol, cinnamon oil, cinnamic aldehyde, and mixtures thereof.

The products comprising a mixture or a flavour composition according to the present invention are regularly products which are intended to be introduced into the human oral cavity, to remain there for a certain period of time and then either to be consumed (e.g. foodstuffs) or to be removed from the oral cavity again (e.g. chewing gums or toothpaste). In this context, these products include all substances or products which are intended to be taken in by humans in the processed, partly processed or non-processed state. These also include substances which are added to foodstuffs during their production, processing or preparation and are envisaged for introduction into the human oral cavity.

Foodstuffs according to the present invention are for example bakery prod- ucts (for example bread, dry biscuits, cakes, other pastry products), confectionery (for example chocolates, chocolate bar products, other bar products, fruit gums, hard and soft caramels, chewing gum), alcoholic or nonalcoholic beverages (for example coffee, tea, wine, beverages containing wine, beer, beverages containing beer, liqueurs, spirits, brandies, (fruit- containing) carbonated beverages, isotonic beverages, soft drinks, nectars, fruit and vegetable juices, fruit or vegetable juice preparations), instant beverages (for example instant cocoa beverages, instant tea beverages, instant coffee beverages), meat products (for example ham, fresh or cured sausage preparations, spiced or marinated fresh or cured meat products), eggs or egg products (dried egg, egg white, egg yolk), cereal products (for example breakfast cereals, muesli bars, precooked ready rice products), dairy products (for example milk beverages, milk ice cream, yogurt, kefir, curd cheese, soft cheese, hard cheese, dried milk powder, whey, butter, buttermilk), fruit preparations (for example jams, fruit ice cream, fruit sauces, fruit fillings), vegetable preparations (for example ketchup, sauces, dried vegetables, deep-frozen vegetables, precooked vegetables, preserved vegetables), snack articles (for example baked or fried potato chips or potato dough products, maize- or peanut-based extrudates), fat- or oil- based products or emulsions thereof (for example mayonnaise, remoulade, dressings), other ready-to-serve meals and soups (for example dried soups, instant soups, precooked soups), spices, seasoning mixtures and in particular powdered seasonings, which are for example used in snack food applications. The products for the purposes of the invention may also be nutritional supplements in the form of capsules, tablets (uncoated and coated tablets, for example coatings resistant to gastric juices), sugar- coated tablets, granules, pellets, mixtures of solids, dispersions in liquid phases, as emulsions, as powders, as solutions, as pastes or as other swallowable or chewable preparations.

An oral care product (also called oral hygiene product) in the context of the invention is understood as meaning one of the formulations familiar to the person skilled in the art for cleansing and care of the oral cavity and the pharyngeal cavity and for refreshing the breath. This expressly includes care of the teeth and gums. Presentation forms of the usual oral hygiene formulations are creams, gels, pastes, foams, emulsions, suspensions, aerosols and sprays, and also capsules, granules, pastilles, tablets, candies or chewing gums, without this list being intended to be understood as limiting for the purpose of this invention.

Preferred oral care products are in particular dental care products such as toothpastes, dental creams, dental gels, dental powders, tooth-cleaning liquids, tooth-cleaning foams, mouthwashes, dental cream and mouthwash as a 2-in-1 product, sugar-free candies for sucking, oral sprays, dental floss or dental care chewing gums.

Dental care products (as an example of the group of oral care products) which contain a mixture according to the invention generally comprise an abrasive system (abrasive or polishing agent), such as for example silicas, calcium carbonates, calcium phosphates, e.g. calcium-glycerophosphates, the Ca-salt of glycerol-1 -phosphoric acid, glycerol-2-phosphoric acid or glycerol-3-phosphoric acid, or mixtures thereof, aluminum oxides and/or hydroxyapatites, surface-active substances such as for example sodium lauryl sulfate, sodium lauryl sarcosinate and/or cocamidopropyl betaine, humectants such as for example glycerol and/or sorbitol, thickeners, such as for example carboxymethylcellulose, polyethylene glycols, carrageenan and/or Laponite^{(R)}, sweeteners, such as for example saccharin, sodium cyclamate, sucralose, acesulfame-K or sugar alcohol flavour-correcting agents for unpleasant flavour impressions such as for example hydroxyfla- vanones according to US 2002/0188019, flavour-correcting agents for further, generally not unpleasant flavour impressions, flavour-modulating substances (for example inositol phosphate, nucleotides such as guanosine monophosphate, adenosine monophosphate or other substances such as sodium glutamate or 2-phenoxypropionic acid), cooling active ingredients, stabilizers and active ingredients, such as for example sodium fluoride, sodium monofluorophosphate, tin difluoride, quaternary ammonium fluorides, zinc citrate, zinc sulfate, tin pyrophosphate, tin dichloride, blends of different pyrophosphates, triclosan (2,4,4'-trichlor-2'-hydroxydiphenyl ether), cetylpyridinium chloride, aluminum lactate, potassium citrate, potassium nitrate, potassium chloride, potassium oxalate, strontium chloride, hydrogen peroxide, aromas and/or sodium bicarbonate or flavour-correcting agents.

Chewing gums (as a preferred example of the group of oral care products or confectionery) which contain a mixture according to the invention generally comprise a chewing gum base, i.e. a chewable mass which becomes plastic on chewing, sugars of various kinds, sugar substitutes, other sweet- tasting substances, sugar alcohols, flavour-correcting agents for unpleasant flavour impressions, other flavour modulators for further, generally not unpleasant flavour impressions, flavour-modulating substances (for example inositol phosphate, nucleotides such as guanosine monophosphate, adenosine monophosphate or other substances such as sodium glutamate or 2-phenoxypropionic acid), humectants, thickeners, emulsifiers, aromas and stabilizers or flavour-correcting agents.

Preferably the products in accordance with the present invention include one or more sweet-tasting substances (including natural sources of these substances), such as for example sweet-tasting carbohydrates or sugars (e.g. sucrose (synonymous with saccharose), trehalose, lactose, maltose, melizitose, raffinose, palatinose, lactulose, D-fructose, D-glucose, D- galactose, L-rhamnose, D-sorbose, D-mannose, D-tagatose, D-arabinose, L-arabinose, D-ribose, D-glyceraldehyde, maltodextrin) or vegetable prepa- rations containing predominantly these carbohydrates (e.g. from sugar beet (Beta vulgaris ssp., sugar fractions, sugar syrup, molasses), from sugar cane [Saccharum officinarum ssp., e.g. molasses, sugar syrups), from sugar maple {Acer ssp.), from agave (agave thick juice), syn- thetic/enzymatic hydrolysates of starch or sucrose (e.g. invert sugar syrup, highly enriched fructose syrups made from corn starch), fruit concentrates (e.g. from apples or pears, apple syrup, pear syrup), sugar alcohols (e.g. erythritol, threitol, arabitol, ribitol, xylitol, sorbitol, mannitol, dulcitol, lactitol), proteins (e.g. miraculin, monellin, thaumatin, curculin, brazzein), sweeten- ers (magap, sodiumcyclamate, acesulfame K, neohesperidin dihydrochal- cone, saccharin sodium salt, aspartame^{(R)}, superaspartame, neotame, ali- tame, sucralose, stevioside, rebaudioside, lugduname, carrelame, su- crononate, sucrooctate, monatin, phyllodulcin), certain sweet-tasting amino acids (glycine, D-leucine, D-threonine, D-asparagine, D-phenylalanine, D- tryptophan, L-proline), other sweet-tasting low-molecular substances (e.g. hernandulcin, dihydrochalcone glycosides, glycyrrhizin, glycyrrhetinic acid ammonium salt or other glycyrrhetinic acid derivatives), liquorice extracts (Glycyrrhizza glabra ssp.), Lippia dulcis extracts, Momordica ssp. extracts or individual substances (in particular Momordica grosvenori [Luo Han Guo] and the mogrosides obtained therefrom), Hydrangea dulcis or Stevia ssp. (e.g. Stevia rebaudiana) extracts or individual substances.

A suitable confectionery sugar base, e.g. for a hard candy, generally comprises from about 30 % to about 85 % glucose syrup and from about 15 % to about 70 % sucrose. Alternatively, a sugar-free base can be used for the shell.

Suitable sugar-free bases, e.g. for confectionery and oral care products, generally include bulk sweeteners such as isomalt, maltitol, sorbitol and xylitol. Isomalt and maltitol are preferred as bulk sugar-free bases. Xylitol is preferred as an ancillary base, preferably being present in sugar-free can- dies at a level of from about 0.1 % to about 5 %.

Products of the present invention may comprise sweeteners, preferably selected from the group saccharin, sodium cyclamate, sucralose, acesulfame-K and aspartame.

Combinations of a mixture according to the present invention and one or more sugar alcohols selected from the group consisting of xylitol, sorbitol, mannitol and erythritol showed enhanced soothing and cooling effects.

Suitable confectionery forms include hard boiled sweets, soft boiled sweets, chewing gums, gummy-based sweets, centre-fill confectionery, or lollies. The confectionery compositions of the present invention preferably take the form of a hard boiled sweet.

Medicaments according to the invention which are preferred for the purposes of the invention are oral preparations, which for example have the form of capsules, tablets (uncoated and coated tablets, for example coatings resistant to gastric juices), sugar-coated tablets, granules, pellets, mixtures of solids, dispersions in liquid phases, as emulsions, as powders, as solutions, as pastes or as other swallowable or chewable preparations and are used as prescription-only, drugstore-only or other medicaments or as nutritional supplements.

Preferably a medicament of the present invention has the form of confectionery, chewing gum, throat and cough lozenge, throat disc or cough syrup. Preferably the medicament is in the form of a confectionery. In a preferred embodiment the medicaments of the present invention are in the form of a hard boiled candy or gum based confectionery. Hard boiled candies are sugar- or sugar substitute-based compositions wherein the base is formed into a candy mass with cooking and subsequently formed into a drop and allowed to cool. The candy mass once cooled generally forms a glassy matrix that contains the mixture or flavouring composition according to the present invention therein. Once formed, the hard boiled candies preferably have a water level of about 0.1 % to about 4 % by weight. Gum based confectionery are soft to semi-solid compositions which are sugar or sugarsubstitute based, wherein a suitable gelling agent is cooked with the sugar or sugarsubstitute and water to achieve the right consistency, and either deposited into moulds or extruded into a continuous rope AND cut.

Other preferred forms of the medicament of the present invention are cough lozenges and cough syrups. Cough lozenges are sugar-based solid or semi-solid compositions, preferably in the form of hard boiled candies and/or gummies. A suitable cough lozenge generally comprises from about

30 % to about 50 % of glucose syrup and from about 15 % to about 75 % of sucrose. Cough lozenges may further comprise honey, honey derivatives and/or honey flavours or lenitive herbs, preferably in concentration from about 0.05 % to 10 %, preferably from 0.1 % to 5 % by weight.

Cough syrups are sugar-based liquid composition further comprising additional active ingredients, such guaifenesin. Cough syrups generally comprise from about 25 % to about 65 % of sucrose. Additionally, from about 30 % to about 45 % of glucose syrup may be further comprised in the formulation of cough syrups.

A medicament of the present invention may further comprise other active ingredients such as local anaesthetics, antitussives, decongestants and the like. The medicaments may also comprise pH adjusting agents and buffers in order to control the pH and hence the stability of the medicament. Other optional ingredients include organic acids including citric, ascorbic, malic, tartaric acids, or mixtures thereof. Where the medicament is the form of a water-based syrup, the medicament may further comprise thickening agents that impart an increased viscosity to the liquid formulation.

Without wishing to be bound by theory it is believed that the mixtures and products of the present invention provide improved soothing of oral and/or nasal tissues by a synergistic action of modulating oral and/or nasal secretions and improved cooling and soothing sensation associated with the cooling agent. The two ingredients act together to provide an improved composition that soothes and relieves irritated oral and/or nasal tissues.

### EXAMPLES

The following examples are given to illustrate the mixtures and uses according to the invention. However, the invention is not limited thereto. Unless indicated otherwise the amounts given are by weight.

### EXAMPLE 1

Pellitorin-blends: Blend A and Blend M (not according to the claims of the present invention)

| | Blend A | Blend M |
|---|---|---|
| Constituent | Proportion in wt.% | Proportion in wt.% |
| Pellitorin* | 10 | 10 |
| Diethyl malonate | 45 | - |
| Peppermint oil (*Mentha arvensis*) | - | 45 |
| Propylene glycol | 45 | 45 |

Pellitorin : a mixture consisting of 94.3 weight percent trans-pellitorin and 4.8 weight percent cis-pellitorin, 0.2 weight percent 2Z,4Z-decadienoic acid-N-isobutylamide and 0.7 weight percent 2Z,4E-decadienoic acid-N-isobutylamide was used.

### EXAMPLE 2

### Flavouring composition Q (a flavouring composition according to the present invention)

| Ingredient | % by weight |
|---|---|
| fractioned Coconut oil | Ad 100 |
| Peppermint oil (*Mentha arvensis*) | 23.80 |
| Peppermint oil (*Mentha piperita*) | 22.00 |
| I-Menthol, | 10.00 |
| d-Limonene | 2.50 |
| Methyl salicylate | 0.50 |
| Vanillin | 1.00 |
| Clove bud oil | 0.70 |
| Neotame | 0.02 |
| Aspartame | 0.04 |
| Sucralose | 1.00 |
| Piperine | 0.05 |
| Frescolat^{®} ML | 2.50 |
| Frescolat^{®} MPC | 1.40 |
| Diethyl tartrate | 2.20 |
| Ethanol | 0.70 |
| Vitamin E - acetate | 0.30 |
| Trans-pellitorin | 0.12 |

Frescolat^{(R)} ML (Symrise): l-menthyl-l-lactate Frescolat^{(R)} MPC (Symrise): I-menthol propylene glycol carbonate

### EXAMPLE 3

### Aroma SP (synthetic peppermint oil) (not according to the present invention)

| | |
|---|---|
| | Aroma SP |
| | parts by weight |
| Isobutyraldehyde | 0.5 |
| 3-Octanol | 0.5 |
| Dimethyl sulfide | 0.5 |
| trans-2-Hexenal | 1.0 |
| cis-3-Hexenol | 1.0 |
| 4-Terpineol, natural | 1.0 |
| Isopulegol | 1.0 |
| Piperitono, natural, from eucalyptus | 2.0 |
| Linalool | 3.0 |
| 8-Ocimenyl acetate, 10 % in triacetin | 5.0 |
| Isoamyl alcohol | 10.0 |
| Isovaleraldehyde | 10.0 |
| alpha-Pinene | 25.0 |
| beta-Pinene, natural | 25.0 |
| Neomenthol, racemic | 40.0 |
| Eucalyptol (1,8-cineol), natural | 50.0 |
| L-Menthyl acetate | 70.0 |
| L-Menthone | 220.0 |
| D-Isomenthone | 50.0 |
| L-Menthol | 484.5 |
| | |
| Total: | 1,000.00 |

### EXAMPLE 4

### Mixture X1 comprising cis- and trans-pellitorin (according to the present invention)

| Constituent | Proportion in wt.% |
|---|---|
| Blend A (of Example 1) | 1 |
| L-Menthol | 5 |
| L-Menthane carboxylic acid N-ethylamide (WS-3, for example Millennium) | 5 |
| L-Menthyl lactate (Frescolat^{®} ML, Symrise) | 35 |
| O-L-Menthyl-O'-(2-hydroxyethyl) carbonate (Frescolat^{®} MGC, Symrise) | 6 |
| Propylene glycol | 48 |

A strongly cooling, virtually flavourless and odorless aroma blend which is liquid at room temperature (20 degrees centigrade) is obtained by blending the constituents.

### EXAMPLE 5

### Mixture X2 comprising cis- and trans-pellitorin (according to the present invention)

| Constituent | Proportion in wt.% |
|---|---|
| Blend M (of Example 1) | 1 |
| Peppermint oil | 15 |
| l-Menthol | 12 |
| L-Menthyl lactate (Frescolat^{®} ML, (Symrise) | 42 |
| N-[[5-methyl-2-(1-methylethyl)cyclohexyl]-carbonyl]glycine ethyl ester (WS-5) | 5 |
| O-L-Menthyl-O'-(2-hydroxyethyl) carbonate (Frescolat^{®} MGC, Symrise) | 10 |
| Propylene glycol | 15 |

A strongly cooling aroma blend with a strong odor of peppermint is obtained by blending the constituents.

### EXAMPLE 6

### Mixture X3 comprising trans-pellitorin (according to the present invention)

| Constituent | Proportion in wt.% |
|---|---|
| Trans-Pellitorin | 0.125 |
| L-Menthyl-3-oxo butyrate | 17.50 |
| L-Menthyl lactate (Frescolat^{®} ML, Symrise) | 40 |
| O-L-Menthyl-O'-(2-hydroxyethyl) carbonate (Frescolat^{®} MGC, Symrise) | 10 |
| Diethyl malonate | 10 |
| Propylene glycol | 22.375 |

A strongly cooling aroma blend which stimulates salivation and causes a tingling effect is obtained by blending the constituents.

### FORMULATION EXAMPLES

### FORMULATION F1

### Transparent tooth gel with capsules

| Ingredients | I (wt.%) | II (wt.%) | III (wt.%) |
|---|---|---|---|
| Sorbitol, 70% strength | Ad 100 | Ad 100 | Ad 100 |
| Distilled water | 11.40 | 11.40 | 11.40 |
| Saccharin | 0.20 | 0.20 | 0.20 |
| Sodium monofluorophosphate | 1.10 | 1.10 | 1.10 |
| Trisodium phosphate | 0.10 | 0.10 | 0.10 |
| Polyethylene glycol PEG 1500 (PEG-32) | 5.50 | 5.50 | 5.50 |
| Abrasive silica gel | 8.00 | 8.00 | 8.00 |
| Thickening silica gel | 8.00 | 8.00 | 8.00 |
| Sodium carboxymethyl cellulose | 0.60 | 0.60 | 0.60 |
| Sodium lauryl sulphate | 1.50 | 1.50 | 1.50 |
| Blend M (of Example 1) | 0.01 | - | - |
| Mixture X1 (of Example 4) | - | 0.80 | - |
| Mixture X2 (of Example 5) | - | - | 0.90 |
| Aroma SP (of Example 3) | 1.00 | 0.60 | 0.20 |
| Blue and red colored microcapsules | 0.50 | 0.80 | 0.70 |
| 4-Hydroxybenzoic acid methylester | 0.10 | 0.10 | 0.10 |

### FORMULATION F2

### Calcium carbonate based toothpaste (pH = 9.6)

| Ingredients | wt.% | wt.% | wt.% |
|---|---|---|---|
| Calcium carbonate | 40.00 | 40.00 | 40.00 |
| Sorbitol | 27.00 | 27.00 | 27.00 |
| Hydrated silica | 2.00 | 2.00 | 2.00 |
| Sodium monofluorophosphate | 0.80 | 0.80 | 0.80 |
| Trisodium phosphate | 0.50 | 0.50 | 0.50 |
| Titanium dioxide | 1.00 | 1.00 | 1.00 |
| Sodium carboxymethyl cellulose | 0.90 | 0.90 | 0.90 |
| Sodium lauryl sulphate | 2.00 | 2.00 | 2.00 |
| Sodium saccharin | 0.20 | 0.20 | 0.20 |
| Sodium fluoride | 0.20 | 0.20 | 0.20 |
| Blend M (of Example 1) | 0.01 | - | - |
| Flavouring composition Q (of Example 2) | - | 1.10 | - |
| Mixture X2 (of Example 5) | - | - | 0.90 |
| Aroma SP (of Example 3) | 1.00 | - | 0.20 |
| Water | Ad 100 | Ad 100 | Ad 100 |

### FORMULATION F3

### Readv-to-use mouthwash composition

| Ingredients | Wt.% | Wt.% |
|---|---|---|
| Ethanol | 7.00 | 7.00 |
| Glycerin | 12.00 | 12.00 |

| | | |
|---|---|---|
| Sodium fluoride | 0.05 | 0.05 |
| Pluronic F-127®(BASF, surfactant) | 1.40 | 1.40 |
| Na-phosphate buffer pH 7,0 | 1.10 | 1.10 |
| Sorbic acid | 0.20 | 0.20 |
| Sodium saccharin | 0.10 | 0.10 |
| Flavouring composition Q (of Example 2) | 0.60 | - |
| Mixture X3 (of Example 6) | - | 0.50 |
| Aroma SP (of Example 3) | - | 0.20 |
| Color FD&C Blue #1 | 0.01 | 0.01 |
| Water | Ad 100 | Ad 100 |

### FORMULATION F4

### Gel dental cream having an activity against bad breath

| | I (wt.%) | II (wt.%) | III (wt.%) |
|---|---|---|---|
| Na-carboxymethylcellulose | 0.40 | 0.40 | 0.40 |
| Sorbitol 70 %, in water | 72.00 | 72.00 | 72.00 |
| Polyethylene glycol (PEG) 1500 | 3.00 | 3.00 | 3.00 |
| Na-saccharinate | 0.07 | 0.07 | 0.07 |
| A-fluoride | 0.24 | 0.24 | 0.24 |
| p-Hydroxybenzoic acid (PHB) ethyl | 0.15 | 0.15 | 0.15 |

| | | | |
|---|---|---|---|
| ester | | | |
| Abrasive silica | 11.00 | 11.00 | 11.00 |
| Thickening silica | 6.00 | 6.00 | 6.00 |
| Triclosan (2,4,4'-trichlor-2'-hydroxydiphenyl ether) | - | 0.30 | 0.30 |
| Blend A (of Example 1) | 0.01 | - | - |
| Flavouring composition Q (of Example 2) | - | 1.10 | - |
| Mixture X3 (of Example 6) | - | - | 0.80 |
| Aroma SP (of Example 3) | 0.90 | - | 0.20 |
| Menthone glycerine acetal (Frescolat^{®} MGA) | 0.10 | 0.08 | - |
| Sodium dodecyl sulfate (SDS) | 1.40 | 1.40 | 1.40 |
| Dist. water | Ad 100.00 | Ad 100.00 | Ad 100.00 |

### FORMULATION F5

### Dental cream against plaque

| | I (wt.%) | II (wt.%) | III (wt.%) |
|---|---|---|---|
| Na-carboxymethylcellulose | 1.00 | 1.00 | 1.00 |
| Glycerol | 12.50 | 12.50 | 12.50 |

| | | | |
|---|---|---|---|
| Sorbitol 70 %, in water | 29.00 | 29.00 | 29.00 |
| Na-saccharinate | 0.20 | 0.20 | 0.20 |
| Na-fluoride | 0.22 | 0.22 | 0.22 |
| Azacycloheptane-2,2-diphosphoric acid, di-sodium salt | 1.00 | 1.00 | 1.00 |
| Bromochlorophene | 0.10 | 0.10 | 0.10 |
| Abrasive silica (Sident 9, Degussa) | 15.00 | 15.00 | 15.00 |
| Thickening silica (Sident 22, Degussa) | 5.00 | 5.00 | 5.00 |
| Flavouring composition Q (of Example 2) | 1.05 | - | - |
| Mixture X1 (of Example 4) | - | 1.00 | |
| Mixture X2 (of Example 5) | - | - | 0.80 |
| Aroma SP (of Example 3) | - | 0.75 | 0.35 |
| Sodium dodecyl sulfate (SDS) | 1.50 | 1.50 | 1.50 |
| Dist. water | Ad 100.00 | Ad 100.00 | Ad100.00 |

### FORMULATION F6

**Dental cream against plaque**

| | I (wt.%) | II (wt.%) | III (wt.%) |
|---|---|---|---|
| Carrageenan | 0.90 | 0.90 | 0.90 |
| | | | |
| Glycerol | 15.00 | 15.00 | 15.00 |
| Sorbitol 70 %, in water | 25.00 | 25.00 | 25.00 |
| PEG 1000 | 3.00 | 3.00 | 3.00 |
| Na-fluoride | 0.24 | 0.24 | 0.24 |
| Tetrapotassium diphosphate | 4.50 | 4.50 | 4.50 |
| Tetrasodium diphosphate | 1.50 | 1.50 | 1.50 |
| Na saccharinate | 0.40 | 0.40 | 0.40 |
| Precipitated silica | 20.00 | 20.00 | 20.00 |
| Titanium dioxide | 1.00 | 1.00 | 1.00 |
| p-Hydroxybenzoic acid methyl ester | 0.10 | 0.10 | 0.10 |
| Flavouring composition Q (of Example 2) | 1.05 | - | - |
| Mixture X1 (of Example 4) | - | 0.90 | - |
| Mixture X3 (of Example 6) | - | - | 0.65 |
| Aroma SP (of Example 3) | - | 0.80 | 0.70 |
| Sodium dodecyl sulfate | 1.30 | 1.30 | 1.30 |
| Dist. water | Ad 100.00 | Ad 100.00 | Ad 100.00 |

### FORMULATION F7

### Dental cream against sensitive teeth

| | I (wt.%) | II (wt.%) | III (wt.%) |
|---|---|---|---|
| Na-carboxymethylcellulose | 0.70 | 0.70 | 0.70 |
| Xanthan Gum | 0.50 | 0.50 | 0.50 |
| Glycerol | 15.00 | 15.00 | 15.00 |
| Sorbitol 70 %, in water | 12.00 | 12.00 | 12.00 |
| K-nitrate | 5.00 | 5.00 | 5.00 |
| Na-monofluorophosphate | 0.80 | 0.80 | 0.80 |
| p-Hydroxybenzoic acid methyl ester | 0.15 | 0.15 | 0.15 |
| p-Hydroxybenzoic add propyl ester | 0.05 | 0.05 | 0.05 |
| Na saccharinate | 0.20 | 0.20 | 0.20 |
| Wintergreen aroma (contains methyl salicylate) | 0.80 | 0.15 | 0.60 |
| Mixture X1 (of Example 4) | 0.90 | - | - |
| Mixture X2 (of Example 5) | - | 1.10 | - |
| Mixture X3 (of Example 6) | - | - | 0.90 |
| Ca-carbonate | 35.00 | 35.00 | 35.00 |
| Silicon dioxide | 1.00 | 1.00 | 1.00 |
| Sodium dodecyl sulfate (SDS) | 1.50 | 1.50 | 1.50 |
| Dist. water | Ad 100.00 | Ad 100.00 | Ad 100.00 |

### FORMULATION F8

### Mouthwash concentrate

| | I (wt.%) | II (wt.%) | III (wt.%) |
|---|---|---|---|
| Ethanol, 95 % strength | 80.00 | 80.00 | 80.00 |
| Na cyclamate | 0.15 | 0.15 | 0.15 |
| Eucalyptol aroma (contains natural eucalyptol) | 1.00 | 1.00 | 1.00 |
| Dyestuff | 0.01 | 0.01 | 0.01 |
| Flavouring composition Q (of Example 2) | 2.50 | 3.80 | - |
| Mixture X2 (of Example 5) | - | - | 3.00 |
| Dist. water | Ad 100.00 | Ad 100.00 | Ad 100.00 |

### FORMULATION F9

### Sugar-containing chewing gum

| | I (wt.%) | II (wt.%) | III (wt.%) |
|---|---|---|---|
| Chewing gum base | 21.00 | 21.00 | 21.00 |
| Glucose syrup | 16.50 | 16.50 | 16.50 |
| Glycerol | 0.50 | 0.50 | 0.50 |
| Powdered sugar | Ad 100 | Ad 100 | Ad 100 |
| Encapsulated wintergreen aroma (contains methyl salicylate) | - | 0.10 | 0.80 |
| Flavouring composition Q (of Example 2) | 1.10 | - | - |
| Mixture X1 (of Example 4) | - | 1.00 | 1.00 |
| Aroma SP (of Example 3) | - | 0.70 | - |

### FORMULATIONS F10a - 10d: Sugar-free chewing gums

### FORMULATION F10a

### Non-stick chewing gum

Chewing gum base K1 comprised 2.0 percent butyl rubber (isobutene/isoprene copolymer, MW 400,000), 6.0 percent polyisobutene (MW = 43,800), 43.5 percent polyvinyl acetate (MW 12,000), 31.5 percent polyvinyl acetate (MW = 47,000), 6.75 percent triacetin and 10.25 percent calcium carbonate. Chewing gum base K1 and the chewing gums can be prepared analogously to US 5,601,858.

| | I (wt.%) | II (wt.%) | III (wt.%) |
|---|---|---|---|
| Chewing gum base K1 | 26.00 | 26.00 | 26.00 |
| Triacetin | 0.25 | 0.25 | 0.25 |
| Lecithin | 0.50 | 0.50 | 0.50 |
| Sorbitol, crystalline | Ad 100 | Ad 100 | Ad 100 |
| Mannitol | 15.30 | 15.20 | 15.10 |
| Glycerol | 12.10 | 12.00 | 11.80 |
| Aspartame | 0.17 | 0.17 | 0.17 |
| Encapsulated aspartame | 1.08 | 1.08 | 1.08 |
| Amorphous silica | 1.00 | 1.00 | 1.00 |
| Cottonseed oil | 0.50 | 0.50 | 0.50 |
| Polyoxyethylene sorbitan monolaurate (E-432) | 1.00 | 1.00 | 1.00 |
| Menthone glycerine acetal (Frescolat^{®} MGA) | - | 0.15 | - |
| Encapsulated spearmint aroma (contains I-carvone) | 0.20 | 0.10 | 0.35 |
| Encapsulated wintergreen aroma (contains methyl salicylate) | - | 0.10 | - |

| | | | |
|---|---|---|---|
| Flavouring composition Q (of Example 2) | 1.30 | 1.10 | - |
| Mixture X2 (of Example 5) | - | - | 1.20 |

### FORMULATION F10b

### Bubble gum

The bubble gum can be prepared analogously to US 5,093,136.

| | I (wt.%) | II (wt.%) |
|---|---|---|
| Styrene/butadiene copolymer (SBR) | 19.50 | 17.50 |
| Polyisobutene | 8.00 | 8.00 |
| Sorbitol powder | Ad 100 | Ad 100 |
| Sorbitol, 70 %, in water | 9.20 | 22.20 |
| Hydrogenated starch hydrolysates (HSH) | 9.00 | - |
| Glycerol | 3.00 | 2.00 |
| Aspartame | 0.10 | 0.10 |
| Encapsulated aspartame | 0.50 | 0.50 |
| Green and blue dyestuff | 0.01 | 0.01 |
| Blend A (of Example 1) | 0.01 | - |
| Cherry-almond aroma | 1.20 | - |
| Mixture X2 (of Example 5) | - | 1.10 |

The chewing gums of recipe (I) were shaped as compact balls, and those of recipe (II) were shaped as hollow balls.

### FORMULATION F10c

Chewing gum base K2 comprised 28.5 percent terpene resin, 33.9 percent polyvinyl acetate (MW = 14,000), 16.25 percent hydrogenated plant oil, 5.5 percent mono- and di-glycerides, 0.5 percent polyisobutene (MW 75,000), 2.0 percent butyl rubber (isobutene/isoprene copolymer), 4.6 percent amorphous silicon dioxide (water content approx. 2.5 percent), 0.05 percent antioxidant tert-butylhydroxytoluene (BHT), 0.2 percent lecithin, and 8.5 percent calcium carbonate. Chewing gum base K2 and the chewing gums can be prepared analogously to US 6,986,907.

| | I (wt.%) | II (wt.%) | III (wt.%) |
|---|---|---|---|
| Chewing gum base K2 | 25.30 | 27.30 | 26.30 |
| Sorbitol | Ad 100 | Ad 100 | Ad 100 |
| Glycerol | 2.40 | 2.40 | 2.40 |
| Lecithin | 7.00 | 7.00 | 7.00 |
| Aspartame | 0.14 | 0.14 | 0.14 |
| Encapsulated aspartame | 0.68 | 0.68 | 0.68 |
| Menthol, spray-dried | 0.25 | 0.10 | 0.50 |
| Lemon aroma, spray-dried | - | 1.20 | - |
| Flavouring composition Q (of Example 2) | 1.25 | - | - |
| Mixture X1 (of Example 4) | - | 1.20 | - |
| Mixture X2 (of Example 5) | - | - | 0.95 |

The chewing gums of recipe (I) and (II) were shaped as strips, and those of recipe (III) were shaped as pellets.

### FORMULATION F10d

| | I (wt.%) | II (wt.%) | III (wt.%) |
|---|---|---|---|
| Chewing gum base | 30.00 | 30.00 | 30.00 |
| Sorbitol, powder | Ad 100 | Ad 100 | Ad 100 |
| Palatinite | 9.50 | 9.50 | 9.50 |
| Xylitol | 2.00 | 2.00 | 2.00 |
| Mannitol | 3.00 | 3.00 | 3.00 |
| Aspartame | 0.10 | 0.10 | 0.10 |
| Acesulfame K | 0.10 | 0.10 | 0.10 |
| Emulgum / emulsifier | 0.30 | 0.30 | 0.30 |
| Sorbitol 70 %, in water | 14.00 | 14.00 | 14.00 |
| Glycerol | 1.00 | 1.00 | 1.00 |
| Flavouring composition Q (of Example 2) | 1.05 | - | - |
| Mixture X1 (of Example 4) | - | 0.90 | - |
| Blend M (of Example 1) | - | - | 0.01 |
| Aroma SP (of Example 3) | - | 0.80 | 1.20 |

| Core composition: | | | |
|---|---|---|---|
| Plant oil triglyceride (coconut oil fraction) | 80.73 | 68.80 | 56.65 |
| Aroma Q (of Example 2) | - | 30.0 | 20.50 |
| Mixture X1 (of Example 4) | - | - | 1.25 |
| Mixture X3 (of Example 6) | 1.00 | - | - |
| Aroma SP (of Example 3) | 18.00 | - | 20.00 |
| Neotame and aspartame | - | 0.05 | - |
| Sucralose | 0.22 | 0.30 | 0.70 |
| (1 R,3R,4S) Menthyl-3-carboxylic acid N-ethylamide (WS-3) | - | 0.55 | - |
| (-)-Menthone glycerol acetal (Frescolat^{®} MGA) | - | 0.30 | 0.80 |
| Vanillin | 0.05 | - | 0.10 |

### FORMULATION F11

### Gelatine capsule for direct consumption

| | I (wt.%) | II (wt.%) | III (wt.%) |
|---|---|---|---|
| Gelatine shell: | | | |
| Glycerol | 2.014 | 2.014 | 2.014 |
| Gelatine 240 Bloom | 7.91 | 7.91 | 7.91 |
| Sucralose | 0.065 | 0.065 | 0.065 |
| Allura Red | 0.006 | 0.006 | 0.006 |
| Brilliant Blue | 0.005 | 0.005 | 0.005 |
| | | | |

| Core composition: | | | |
|---|---|---|---|
| Plant oil triglyceride (coconut oil fraction) | 80.73 | 68.80 | 56.65 |
| Aroma Q (of Example 2) | - | 30.0 | 20.50 |
| Mixture X1 (of Example 4) | - | - | 1.25 |
| Mixture X3 (of Example 6) | 1.00 | - | - |
| Aroma SP (of Example 3) | 18.00 | - | 20.00 |
| Neotame and aspartame | - | 0.05 | - |
| Sucralose | 0.22 | 0.30 | 0.70 |
| (1R,3R,4S) Menthyl-3-carboxylic acid N-ethylamide (WS-3) | - | 0.55 | - |
| (-)-Menthone glycerol acetal (Frescolat^{®} MGA) | - | 0.30 | 0.80 |
| Vanillin | 0.05 | - | 0.10 |

The gelatine capsule, which is suitable for direct consumption, was prepared in accordance with WO 2004/050069 and had a diameter of 5 mm, and the weight ratio of core material to shell material was 90: 10. The capsules opened in the mouth within less than 10 seconds and dissolved completely within less than 50 seconds.

### FORMULATION F12

### Chewing candy with aroma according to the invention

| | | |
|---|---|---|
| Water | | 7.8 % |
| Sugar | *refined sugar C4* | Ad 100 |
| Glucose syrup | dextrose 40 | 37.3 % |
| Hydrogenated plant fat | melting point 32-36 °C | 6.6 % |
| Lecithin | emulsifier (soya lecithin) | 0.3 % |
| Gelatine | pig gelatine | 0.8 % |
| Fondant | type S30 | 4.9 % |
| Mixture X2 (of Example 5) | | 0.9 % |
| (-)-Menthane glycerol acetal | Frescolat^{®} MGA | 0.2 % |

### FORMULATION F13

### Compressed tablets with aroma according to the invention

| | |
|---|---|
| Dextrose | Ad 100 |
| Magnesium stearate (lubricant) | 0.9 wt.% |
| Citric acid | 0.3 wt.% |
| Mixture X1 (of Example 4) | 0.8 wt.% |
| Orange-flavour | 0.25 wt.% |

### FORMULATION F14

### Extrudate in the glassy state

| | | Wt.% |
|---|---|---|
| Glucose syrup, spray-dried (DE value: 31-34) | Glucidex IT33W (Roquette) | 61.0 |
| Maltodextrin (DE value: 17-20) | (Cerestar) | 28.4 |
| Emulsifler Monomuls | emulsifler based on hydrogenated palm oil; melting point: 64°C | 1.8 |
| Dextrose monohydrate (DE value: 99.5) | dextrose, containing water of crystallization (Cerestar) | 1.8 |
| Water | | 2.0 |
| Mixture X1 (of Example 4) | | 1.2 |
| Aroma SP (of Example 3) | | 3.8 |

### Preparation instructions (see also WO 03/092412):

All the constituents were mixed and the mixture was conveyed in a twin- screw extruder by one-point metering. The extrusion temperatures were between 100 and 120 degrees centigrade and the specific energy input was 0.2 kWh/kg. The strands emerging from the extruder die plate, which was provided with

1 mm bores, were cut to particles of approx. 1 mm diameter by rotating blades immediately after exit from the dies.

### FORMULATION F15

### Fluidized bed granules with aroma according to the invention

A solution consisting of 44 weight percent water, 11 weight percent Mixture X1 (according to the invention from Example 4), 13 weight percent gum arabic and 32 weight percent hydro- lysed starch (maltodextrin DE 15-19) and some green dyestuff is granulated in a granulating apparatus of the type described in EP 163 836 (with the following features: diameter of in-flow tray 225 mm, spray nozzle: two- component nozzle; sifting discharge: zigzag sifter; filter: internal bag filter). The solution is sprayed into the fluidized bed granulator at a temperature of 32 degrees centigrade. To fluidize the contents of the bed, an amount of 140 kg/h of nitrogen is blown in. The entry temperature of the fluidizing gas is 140 degrees centigrade. The temperature of the waste gas is 76 degrees centigrade. Nitrogen is likewise fed in as the sifting gas in an amount of 15 kg/h at a temperature of 50 degrees centigrade. The contents of the fluidized bed is approx. 500 g. The granulation output is approx. 2.5 kg per hour. Free-flowing granules having an average particle diameter of 360 micrometres are obtained. The granules are circular and have a smooth surface. On the basis of the constant pressure loss of the filter and the content of the bed, which likewise remains constant, stationary conditions are to be assumed in respect of the granulation process.

### FORMULATION F16

### Chewing gum dragees, sugar-free

### Q1: Chewing gum base composition constituents

| | I (wt.%) | II (wt.%) | III (wt.%) |
|---|---|---|---|
| Chewing gum base | 37.00 | 37.00 | 37.00 |
| Sorbitol, powder | Ad 100 | Ad 100 | Ad 100 |
| Aspartame | 0.20 | 0.20 | 0.20 |
| Plasticizer (Emulgum) | 0.50 | 0.50 | 0.50 |
| Acesulfame K | 0.20 | 0.20 | 0.20 |
| Sorbitol 70 % strength in water | 5.00 | 5.00 | 5.00 |
| Glycerol | 4.00 | 4.00 | 4.00 |
| Mixture X1 (of Example 4) | 1.00 | - | |
| Mixture X2 (of Example 5) | - | 1.00 | - |
| Mixture X3 (of Example 6) | - | - | 0.90 |
| Spearmint-Flavour | - | - | 0.50 |
| Menthol, crystalline | 0.80 | - | 0.20 |
| Menthol, spray-dried | - | 0.50 | 0.50 |

### Q2: Coating constituents (covering)

(the weight contents stated relate to the total weight of the coating (Q2) applied to the chewing gum pads (Q1); the total weight of Q2 was about 35 percent, based on the weight of Q1)

| | I (wt.%) | II (wt.%) | III (wt.%) |
|---|---|---|---|
| Mixture A | | | |
| Isomalt | 0.20 | - | - |
| Sorbitol | - | 0.40 | - |
| Mannitol | - | - | 0.80 |
| I-Menthol, spray-dried | 0.20 | 0.60 | 0.80 |
| Mixture B | | | |
| Isomalt | 68.00 | 67.70 | 67.40 |
| Water | Ad 100 | Ad 100 | Ad 100 |
| Gum arabic 40 % strength in water (this content includes the amount used for the gumming) | 2.50 | 2.50 | 2.50 |
| Acesulfame K | 0.05 | 0.05 | 0.05 |
| Aspartame | 0.05 | 0.05 | 0.05 |
| Titanium dioxide | 1.50 | 1.50 | 1.50 |
| Constituent C | | | |
| Mixture X1 (of Example 4) | 0.95 | 0.85 | 0.60 |

All the constituents of the chewing gum base composition (Q1) were mixed, stamped into chewing gum strands and then shaped into individual chewing gum pads. The chewing gum pads were then wetted (gummed) with a 40 wt. percent strength gum arabic solution in a rotating dragee-coating drum. The gummed chewing gum pads were then coated in a rotating dragee-coating drum with the pulverulent mixture A, which substantial-ly comprised spray- dried I-menthol and at least one sugar substitute (usually chosen from isomalt, sorbitol, xylitol, maltitol and/or mannitol, pulverulent gum arabic can optionally additionally be used).

After adequate drying with cold air, the chewing gum pads coated in this way were dried overnight. For further application of the coating to the dried, coated chewing gum pads using coating solution B, 15 layers were first applied by means of dragee-coating, and in the 16th layer a mixture of constituent C and mixture B was applied. Thereafter, further layers were applied using mixture B, until the total weight of the coating (Q2) was about 35 weight percent of the weight of the original chewing gum pads (Q1). In order to impart gloss to the chewing gum dragees, a final treatment was carried out with a polishing agent, which comprised a mixture of equal weight contents of carnauba wax and bees-wax.

### FORMULATION F17

### Spray-dried aroma according to the invention

A spray-dried aroma according to the invention colored green-yellow (comprising maltodextrin (DE: 18-20), dextrose, gum arabic, Mixture X2 (of Example 5), dyestuff and the antioxidant ascorbyl palmitate) with the following particle size distribution was prepared via a pressure nozzle:
D (v 0.1): 26.8 micrometres,
D (v 0.5): 68.02 micrometres,
D (v 0.9): 126.4 micrometres

### FORMULATION F18

### Instant drink powder with spray-dried aroma

| | |
|---|---|
| Sugar (sucrose) | Ad 100 |
| Citric acid | 11.58 wt.% |
| Trisodium citrate | 0.70 wt.% |
| Tricalcium phosphate | 0.60 wt.% |
| Vitamin C | 0.66 wt.% |
| Grindsted^{®} JU 543 Stabilizer System (Danisco) | 0.90 wt.% |
| Saccharin | 0.561 wt.% |
| Lemon aroma, spray-dried | 1.75 wt.% |
| Aroma, spray-dried, according to Example F17 | 1.50 wt.% |

45 g of this instant drink powder were dissolved in 1,000 ml, while stirring. The drink obtained had a refreshing, cooling flavour of lemon and menthol- peppermint.

### FORMULATION F19

### Hard caramel (hard boiled candy)

| | I (wt.%) | II (wt.%) |
|---|---|---|
| Sugar (sucrose) | Ad 100 | Ad 100 |
| Maize syrup (com syrup), contains glucose and fructose | 41.00 wt.% | 41.00 wt.% |
| Maltose | 3.00 wt.% | 3.00 wt.% |
| Palm kernel oil | 0.90 wt.% | 0.90 wt.% |
| Citric acid | 0.30 wt.% | 0.30 wt.% |
| Ginseng extract | 0.40 wt.% | 0.40 wt.% |
| Blue dyestuff | 0.01 wt.% | 0.01 wt.% |
| Mixture X2 (of Example 5) | 1.30 wt.% | - |
| Honey | - | 1.50 wt.% |
| Honey flavour | - | 0.30 wt.% |
| Mixture X3 (of Example 6) | - | 0.90 wt.% |

The sugar, corn syrup and maltose were dissolved in water and the solution was boiled and placed under a vacuum. The remaining ingredients were then sucked into the boiled sugar mass and the mixture was homogenized at the boiling temperature. After cooling, hard caramels were stamped out of the resulting mass. The hard caramels showed a residual water content of about 2.5 weight percent.

### FORMULATION F20

### Throat candies with a liquid-viscous core filling (centre-filled hard candy)

| | I (wt.%) | II (wt.%) |
|---|---|---|
| Part A (shell) (80 % of the candy) | | |
| Sugar (sucrose) | Ad 100 | Ad 100 |
| Glucose syrup (solids content 80 %) | 41.51 | 49.37 |
| Mixture X1 (of Example 4) | 0.75 | 0.95 |
| I-Menthol | 0.10 | - |
| Lemon oil | 0.10 | 0.10 |
| Citric acid | - | 0.91 |
| Total A: | 100 | 100 |
| Part B (core) (20% of the candy) | | |
| High fructose com syrup (content of solid sugars 85 %, close to 15 % water) | Ad 100 | Ad 100 |
| Glycerol | 15.0 | 15.0 |
| Lecithin | 0.02 | 0.02 |
| Cinnamon oil | - | 0.32 |
| Mixture X2 (of Example 5) | 0.28 | - |
| Capsaicin | 0.05 | - |
| Vanillyl alcohol n-butyl ether | - | 0.10 |
| Red dyestuff, as a 5 % strength aqueous solution | 0.20 | 0.20 |
| Vanillin | 0.07 | - |
| Total B: | 100 | 100 |

Bonbons having a liquid-viscous core were prepared in accordance with the processes described in US 6,432,441 (Example 1 there) and in US 5,458,894 and US 5,002,791. The two parts A and B were processed separately to bases for the shell (Part A) and core (Part B). The filled throat candies obtained by means of co-extrusion acted against coughing, sore throat and hoarseness when consumed by affected persons.

### FORMULATION F21

### Fruit gums

| | I (wt.%) | II (wt.%) |
|---|---|---|
| Water | Ad 100 | Ad 100 |
| Saccharose | 34.50 | 8.20 |
| Glucose syrup, DE 40 | 31.89 | 30.09 |
| Iso Syrup C* Tru Sweet 01750 (Cerestar GmbH) | 1.50 | 2.10 |
| Gelatine 240 Bloom | 8.20 | 9.40 |
| Polydextrose (Litesse^{®} Ultra, Danisco Cultor GmbH) | - | 24.40 |
| Yellow and red coloring | 0.01 | 0.01 |
| Citric acid | 0.20 | - |
| Mixture X2 (of Example 5) | 1.00 | - |
| Mixture X3 (of Example 6) | - | 0.90 |
| Aroma SP (of Example 3) | - | 0.40 |

### FORMULATION F22

### Dental cream and mouthwash as a 2-in-1 product

| | I (wt.%) | II (wt.%) |
|---|---|---|
| Sorbitol | 40.00 | 45.00 |
| Glycerin | 20.00 | 20.00 |
| Ethanol | 5.00 | - |
| Water | Ad 100 | Ad 100 |
| Solbrol M, Na-salt (Methylparaben, sodium salt) | 0.15 | 0.15 |
| Na-monofluorphosphate | 0.75 | 0.75 |
| Saccharine | 0.20 | 0.20 |
| Abrasive silica (Sident 9, Degussa) | 20.00 | 20.00 |
| Thickening silica (Sident 22, Degussa) | 2.00 | 2.00 |
| Sodium carboxymethyl cellulose | 0.30 | 0.30 |
| Sodium lauryl sulfate (SLS) | 1.20 | 1.20 |
| Color (1% in water) | 0.50 | 0.50 |
| Flavouring composition Q (of Example 2) | 1.20 | - |
| Mixture X2 (of Example 5) | - | 1.00 |

### FORMULATION F23

### Flavoured water

| | I (wt.%) | II (wt.%) |
|---|---|---|
| Apple Juice Concentrate (65 °Brix) | 3.50 | 5.00 |
| Apple-Lime Flavour | 0.20 | - |
| Strawberry Flavour | - | 0.20 |
| Citric acid | 0.05 | 0.02 |
| Color (1% in water) | 0.50 | 0.50 |
| Blend A (of Example 1) | 0.01 | - |
| L-Menthyl lactate (Frescolat^{®} ML, Symrise) | 0.10 | - |
| Mixture X1 (of Example 4) | - | 1.00 |
| L-Menthol | 0.05 | 0.10 |
| Water | Ad 100 | Ad 100 |

### FORMULATION F24

### Cough syrup

| | I (wt.%) | II (wt.%) | III (wt.%) |
|---|---|---|---|
| Sucrose | 36.24 | 36.24 | 35.24 |
| Glucose liquid, 80% | 36.24 | 36.24 | 36.24 |
| Honey | - | 1.00 | 1.00 |
| Glycerol | 8.00 | 8.00 | 10.00 |
| Ethanol | 10.00 | 5.00 | 8.50 |
| Citric acid | 0.50 | 0.30 | 0.40 |
| Sodium benzoate | 0.05 | 0.08 | 0.02 |
| Guaifenesin | - | 1.25 | - |
| Codeine | - | - | 0.125 |
| Promethazine | - | - | 0.20 |
| Lemon oil | - | 0.60 | 0.10 |
| Red or yellow color (1% in water) | 0.50 | 0.50 | 0.50 |
| Flavouring composition Q | 1.30 | - | - |
| (of Example 2) | | | |
| Mixture X3 (of Example 6) | - | 0.80 | - |
| Mixture X1 (of Example 4) | - | - | 0.80 |
| Aroma SP (of Example 3) | - | - | 0.60 |
| Water | Ad 100 | Ad 100 | Ad 100 |

## Claims

1. Mixture comprising:
(a) pellitorin, and
(b) one or more physiological cooling agents selected from the group consisting of:
N-[[5-methyl-2-(1-methylethyl)cyclohexyl]-carbonyl]glycine ethyl ester, menthyl-3- hydroxy butyrate, menthyl-3-oxo butyrate, menthyl-3-oxo pentanoate, menthyl lactate, menthone glycerine acetal, menthol glycol carbonate, menthol propyleneglycol carbonate and menthol glycerin carbonate;
and optionally one or more compounds selected from the groups (c), (d) and/or (e):
(c) menthol and/or peppermint oil or mixtures thereof;
(d) one or more additional physiological cooling agents selected from the group consisting of: N-substituted-p-menthane-3-carboxamides, acyclic tertiary and secondary carboxamides, 3-(I-menthoxy)propan-1,2-diol, monomenthyl glutarate, monomenthyl succinate or its salts,
(e) one or more adjuvants selected from the group consisting of glycerin, ethylene glycol, 1,2-propylene glycol, diethyl malonate and/or dibutyl malonate
with the proviso that
(i) a first mixture comprising (in parts per weight)
| | |
|---|---|
| L-N°-(menthancarboxyl)glycin-N-ethylamid | 15 |
| Solution of 10 wt.-% pellitorin in propyleneglycol/peppermint oil (1:1); | 10 |
| L-Menthyllactate | 65 |
| O-L-Menthyl-O'(2-hydroxyethyl)carbonate | 10 |
and
(ii) a second mixture comprising (in parts per weight)
| | |
|---|---|
| Spearmint oil | 10.57 |
| Peppermint oil piperita (menthol content approx.. 53 % b.w.) | 43.44 |
| Sucralose | 1.73 |
| Saccharin | 2.61 |
| Triethyl citrate | 0.10 |
| L-Menthol, natural | 15.59 |
| Triacetine (glycerol triacetate) | 0.07 |
| Benzyl alcohol | 0.07 |
| Capsicum extract 1 million Scoville | 0.07 |
| Ethanol | 5.03 |
| 2-Hydroxyethyl menthyl carbonate | 3.19 |
| L-Menthyl methyl ether | 3.10 |
| Piperine | 0.01 |
| Cis/trans Pellitorin (20/80) (m/m) | 0.01 |
| Plant oil | 4.50 |
| D-Limonene | 2.16 |
| Anethole | 7.75 |
are excluded.

2. Mixture according to claim 1, wherein the weight ratio of trans-pellitorin: cis-pellitorin is in the range of 40: 1 - 1: 1.

3. Mixture according to any of the previous claims, wherein the menthol of component (c) is d-menthol, I-menthol or a mixture of d- and I-menthol.

4. Mixture according to any of the previous claims, comprising one or more compounds of group (d) in an amount sufficient to improve a soothing effect of the mixture on irritated oral and/or nasal tissue.

5. Mixture according to any of the previous claims, comprising one or more compounds of group (e) in an amount sufficient to improve a soothing effect of the mixture on irritated oral and/or nasal tissue, and/or in an amount sufficient for enhancing a bitterness reducing effect of the mixture.

6. Mixture according to any of the previous claims, comprising 0.02 percent to 2 percent by weight pellitorin, and/or 5 % b.w. to 98 % b.w. of cooling agents of group (b).

7. Flavour composition, comprising
(i) a mixture according to any of claims 1 to 6, and
(ii) one or more additional flavouring agents, said flavouring agents not being part of the mixture (i).

8. Foodstuff, confectionary, oral care or pharmaceutical product, comprising a mixture according to any of claims 1 to 6, and/or a flavour composition according to claim 7.

9. Foodstuff, confectionary, oral care or pharmaceutical product according to claim 8, comprising 0.000055 % b.w. to 0.0095 % b.w. of pellitorin.

10. Use of a mixture according to any of claims 1 to 6
- for providing a soothing effect on irritated oral and/or nasal tissue, and/or
- for providing a bitterness reducing effect, and/or
- for providing a cooling and/or freshening effect in the oral and/or nasal cavity.

11. Use of pellitorin for prolonging a cooling and/or freshening effect of cooling agents of group (b) as defined in claim 1.

## Patentansprüche

1. Mischung umfassend:
(a) Pellitorin, und
(b) ein oder mehrere physiologische Kühlmittel ausgewählt aus der Gruppe bestehend aus:
N-[[(5-methyl-2-(1-methylethyl) cyclohexyl]carbonyl]glycin Ethylester, Menthyl-3-hydroxybutyrat, Menthyl-3-oxo-butyrat, Menthyl-3-oxo pentanoat, Menthyllactat, Menthonglycerin Acetal, Mentholglycolcarbonat, Mentholpropylenglycolcarbonat und Mentholglycerincarbonat;
und gegebenenfalls eine oder mehrere Verbindungen ausgewählt aus den Gruppen (c), (d) und/oder (e):
(c) Menthol und/oder Pfefferminzöl oder Mischungen daraus
(d) ein oder mehrere zusätzliche physiologische Kühlmittel ausgewählt aus der Gruppe bestehend aus: N-substituierten-p-Menthan-3-carboxamiden, azyklischen tertiären und sekundären Carboxamiden, 3-(I-Menthoxy) propan-1, 2-diol, Monomenthylsuccinat oder deren Salzen,
(e) ein oder mehrere Hilfsstoffe ausgewählt aus der Gruppe bestehend aus Glycerin, Ethylenglykol, 1, 2-Propylenglykol, Diethylmalonat und / oder Dibutylmalonat
mit der Maßgabe, dass
(i) eine erste Mischung umfassend (in Gewichtsteilen):
| | |
|---|---|
| L-*N*°-(Menthancarboxyl)glycin-*N*-ethylamid | 15 |
| Lösung aus 10 Gew.-% Pellitorin in Propylenglycol/Pfefferminzöl 1:1 | 10 |
| L-Menthyllactat | 85 |
| O-L-Menthyl-O'-(2-hydroxyethyl)carbonat | 10 |
und
(ii) eine zweite Mischung umfassend (in Gewichtsteilen):
| | |
|---|---|
| Grüne Münze Öl | 10.57 |
| Pfefferminzöl piperita (Mentholgehalt ca. 53 Gew.-%.) | 43.44 |
| Sucralose | 1.73 |
| Saccharin | 2.61 |
| Triethylcitrat | 0.10 |
| L-Menthol, natürliche | 15.59 |
| Triacetin (Glycerintriacetat) | 0.07 |
| Benzylalkohol | 0.07 |
| Capsicum Extrakt 1 Million Scoville | 0.07 |
| Ethanol | 5.03 |
| 2-Hydroxyethyl Menthyl Karbonat | 3.19 |
| L-Menthyl-methylether | 3.10 |
| Piperin | 0.01 |
| Cis/trans Pellitorin (20/80) (m/m) | 0.01 |
| Pflanzenöl | 4.50 |
| D-Limonen | 2.16 |
| Anethol | 7.75 |
ausgenommen sind.

2. Mischung nach Anspruch 1, wobei das Gewichtsverhältnis von trans-Pellitorin: cis-Pellitorin im Bereich von 40: 1 - 1: 1 liegt.

3. Mischung nach einem der vorhergehenden Ansprüche, wobei das Menthol der Komponente (c) D-Menthol, L-Menthol oder eine Mischung aus D- und L-Menthol ist.

4. Mischung nach einem der vorhergehenden Ansprüche, umfassend eine oder mehrere Verbindungen der Gruppe (d) in einer Menge, die ausreichend ist, um die Beruhigungswirkung der Mischung auf gereizte orale und/oder nasale Gewebe zu verbessern.

5. Mischung nach einem der vorhergehenden Ansprüche, umfassend eine oder mehrere Verbindungen der Gruppe (e) in einer Menge, die ausreichend ist, um die Beruhigungswirkung der Mischung auf gereizte orale und/oder nasale Gewebe zu verbessern, und/oder in einer Menge, die ausreichend ist, um die bitter-reduzierende Wirkung der Mischung zu steigern.

6. Mischung nach einem der vorhergehenden Ansprüche, umfassend 0,02 bis 2 Gew.-% Pellitorin, und/oder 5 bis 98 Gew.-% von Kühlmitteln der Gruppe (b).

7. Geschmackskomposition, umfassend
(i) eine Mischung nach einem der Ansprüche 1 bis 6, und
(ii) einen oder mehrere weitere Geschmacksstoffe, die nicht Teil der Mischung (i) sind.

8. Nahrungsmittel, Konfekt, Mundpflegeprodukt oder pharmazeutisches Produkt umfassend eine Mischung nach einem der Ansprüche 1 bis 6, und/oder eine Geschmackskomposition nach Anspruch 7.

9. Lebensmittel, Konfekt, Mundpflegeprodukt oder pharmazeutisches Produkt nach Anspruch 8, umfassend 0,000055 bis 0,0095% Gew.-% Pellitorin.

10. Verwendung einer Mischung nach einem der Ansprüche 1 bis 6
- um eine Beruhigungswirkung auf gereizte orale und/oder nasale Gewebe zu bewirken, und / oder
- um eine bitter-reduzierende Wirkung zu bewirken, und/oder
- um einen Kühl- und/oder Erfrischungseffekt in der Mundhöhle und/oder Nasenhöhle zu bewirken.

11. Verwendung von Pellitorin zur Verlängerung einer Kühl- und/oder Frischewirkung von Kühlmitteln der Gruppe (b), wie in Anspruch 1 definiert.

## Revendications

1. Mélange comprenant :
(a) de la pellitorine, et
(b) un ou plusieurs agents rafraîchissants physiologiques choisis dans le groupe constitué par :
l'éthylester de N-[[5-méthyl-2-(1-méthyléthyl)cyclohexyl]carbonyl]glycine, le 3-hydroxybutyrate de menthyle, le 3-oxobutyrate de menthyle, le 3-oxopentanoate de menthyle, le lactate de menthyle, l'acétal de glycérol et de menthone, le carbonate de glycol et de menthol, le carbonate de propylène glycol et de menthol et le carbonate de glycérol et de menthol ;
et éventuellement un ou plusieurs composés choisis parmi les groupes (c), (d) et/ou (e) :
(c) le menthol et/ou l'essence de menthe poivrée, ou des mélanges de ceux-ci ;
(d) un ou plusieurs autres agents rafraîchissants physiologiques choisis dans le groupe constitué par : les p-menthane-3-carboxamides N-substitués, les carboxamides tertiaires et secondaires acycliques, le 3-(I-menthoxy)propane-1,2-diol, le glutarate de monomenthyle, le succinate de monomenthyle ou ses sels,
(e) un ou plusieurs adjuvants choisis dans le groupe constitué par le glycérol, l'éthylène glycol, le 1,2-propylène glycol, le malonate de diéthyle et/ou le malonate de dibutyle ;
à condition que
(i) un premier mélange comprenant (en parties en poids)
| | |
|---|---|
| L-N°-(menthanecarboxyl)glycine-N-éthylamide | 15 |
| Solution de 10% en poids de pellitorine dans du propylène glycol/essence de menthe poivrée (1:1) | 10 |
| Lactate de L-menthyle | 65 |
| Carbonate de O'-(2-hydroxyéthyl) et de O-L-menthyle | 10 |
et
(ii) un deuxième mélange comprenant (en parties en poids)
| | |
|---|---|
| Essence de menthe verte | 10,57 |
| Essence de menthe poivrée *piperita* (teneur en menthol d'environ 53% en poids) | 43,44 |
| Sucralose | 1,73 |
| Saccharine | 2,61 |
| Citrate de triéthyle | 0,10 |
| L-menthol, naturel | 15,59 |
| Triacétine (triacétate de glycérol) | 0,07 |
| Alcool benzylique | 0,07 |
| Extrait de piment, 1 million sur l'échelle de Scoville | 0,07 |
| Ethanol | 5,03 |
| Carbonate de 2-hydroxyéthyle et de menthyle | 3,19 |
| Ether méthylique de L-menthyle | 3,10 |
| Pipérine | 0,01 |
| Cis/trans pellitorine (20/80) (m/m) | 0,01 |
| Huile végétale | 4,50 |
| D-limonène | 2,16 |
| Anéthole | 7,75 |
soient exclus.

2. Mélange selon la revendication 1, dans lequel le rapport pondéral trans-pellitorine:cis-pellitorine se trouve dans la plage de 40:1-1:1.

3. Mélange selon l'une quelconque des revendications précédentes, dans lequel le menthol du composant (c) est le d-menthol, le l-menthol ou un mélange de d- et l-menthol.

4. Mélange selon l'une quelconque des revendications précédentes, comprenant un ou plusieurs composés du groupe (d) selon une quantité suffisante pour améliorer un effet apaisant du mélange sur du tissu oral et/ou nasal irrité.

5. Mélange selon l'une quelconque des revendications précédentes, comprenant un ou plusieurs composés du groupe (e) selon une quantité suffisante pour améliorer un effet apaisant du mélange sur du tissu oral et/ou nasal irrité, et/ou selon une quantité suffisante pour améliorer un effet de réduction d'amertume du mélange.

6. Mélange selon l'une quelconque des revendications précédentes, comprenant de 0,02% à 2% en poids de pellitorine, et/ou de 5% en poids à 98% en poids d'agents rafraîchissants du groupe (b).

7. Composition aromatisante, comprenant
(i) un mélange selon l'une quelconque des revendications 1 à 6, et
(ii) un ou plusieurs autres agents aromatisants, lesdits agents aromatisants ne faisant pas partie du mélange (i).

8. Produit alimentaire, confiserie, produit de soin oral ou pharmaceutique, comprenant un mélange selon l'une quelconque des revendications 1 à 6, et/ou une composition aromatisante selon la revendication 7.

9. Produit alimentaire, confiserie, produit de soin oral ou pharmaceutique selon la revendication 8, comprenant de 0,000055% en poids à 0,0095% en poids de pellitorine.

10. Utilisation d'un mélange selon l'une quelconque des revendications 1 à 6
- pour fournir un effet apaisant sur du tissu oral et/ou nasal irrité, et/ou
- pour fournir un effet de réduction d'amertume, et/ou
- pour fournir un effet refroidissant et/ou rafraîchissant dans la cavité orale et/ou nasale.

11. Utilisation de pellitorine pour prolonger un effet refroidissant et/ou rafraîchissant des agents rafraîchissants du groupe (b) tels que définis selon la revendication 1.
